# EUROPÄISCHE PATENTSCHRIFT

(11) **EP 3 817 658 B1**
(45) Veröffentlichungstag und Bekanntmachung des Hinweises auf die Patenterteilung: **29.06.2022**
(21) Anmeldenummer: 19736325.2
(22) Anmeldetag: 26.06.2019
(51) Int. Cl.: A61B 5/06

(54) **VORRICHTUNG ZUR LOKALISATION EINES MEDIZINISCHEN IMPLANTATS**
DEVICE FOR LOCATING A MEDICAL IMPLANT
DISPOSITIF DE LOCALISATION D'UN IMPLANT MÉDICAL

(30) Priorität: 06.07.2018 DE 102018211185
(43) Veröffentlichungstag der Anmeldung: 12.05.2021
(73) Patentinhaber: Neuroloop GmbH, 79110 Freiburg (DE)
(72) Erfinder: PLACHTA, Dennis, 79279 Vörstetten (DE); BORETIUS, Tim, 79098 Freiburg (DE); KIMMIG, Fabian, 79110 Freiburg (DE)
(74) Vertreter: Rösler, Uwe
(86) Internationale Anmeldenummer: PCT/EP2019/067006
(87) Internationale Veröffentlichungsnummer: WO 2020/007681

(56) Entgegenhaltungen:
- EP-A1- 1 052 935
- US-A1- 2005 212 515
- US-A1- 2010 109 848
- US-A1- 2017 100 056
- US-A1- 2017 367 616

## Beschreibung

### Technisches Gebiet

Die Erfindung bezieht sich auf eine Vorrichtung zur transkutanen Lokalisation eines intrakorporal, subkutan verorteten medizinischen Implantats, das eine Implantatoberfläche aufweist, an der wenigstens eine erste elektrische Spule angeordnet ist, die wenigstens eine, einen Bereich der Implantatoberfläche umschließende Spulenwicklung aufweist. Zum Zwecke der Lokalisation der Spulenwicklung dient eine extrakorporal handhabbare Einheit, an der wenigstens eine elektrische Spule angebracht ist, die mit einer elektrischen Steuereinheit sowie mit einem Signalmittel verbunden ist und mit deren Hilfe eine möglichst maximale Spulenüberlappung zwischen der intra- und extrakorporalen Spule im Wege einer Induktionserfassung erfassbar ist.

### Stand der Technik

Aus der Druckschrift EP 1 052 935 B1 sind ein System sowie ein Verfahren zum Orten einer implementierbaren Medikamentenabgabevorrichtung zu entnehmen, die unmittelbar unter der Haut implantiert wird. Die Medikamentenabgabevorrichtung umfasst u.a. ein Medikamentenreservoir, zu dessen mehrfachen Wiederbefüllung die Reservoirwand ein Septum vorsieht, das im implantierten Zustand der Hautoberfläche zugewandt orientiert ist. Zum Befüllen eines derartig implantierten Medikamentenreservoirs bedarf es der genauen Kenntnis von Lage und Position des subkutan verorteten Plenums, so dass ein Arzt ohne weitere Patientenbelastung und nur mit einem einzigen Einstich mit Hilfe einer Injektionsnadel durch die Haut das darunter liegende Plenum trifft, um den Befüllungsvorgang mit Hilfe einer Injektionsspritzenvorrichtung vornehmen zu können.

Zum Auffinden der exakten Position des intrakorporal verorteten Septums ist eine das Septum umfassende elektrische Spule implantatseitig vorgesehen, die mit einer innerhalb der Medikamentenabgabevorrichtung untergebrachten elektrischen Energiequelle sowie elektronischen Steuerung verbunden ist und als Sendeantenne betreibbar ist. Mit Hilfe einer extrakorporal, handhabbaren Implantat-Ortungsantennenanordnung, die drei gleich verteilt längs einer Kreislinie angebrachte Luftspulen-Antennen aufweist, gilt es die Position der implantatseitig angebrachten Sendeantenne und somit das von dieser umfassten Septum aufzufinden. Hierzu muss die extrakorporale Implantat-Ortungsantennenanordnung solange an der Patientenhautoberfläche bewegen werden, bis die längs der virtuellen Kreislinie gleichverteilt angeordneten drei Luftspulen-Antennen der Ortungsantennenanordnung konzentrisch zu der implantatseitigen Sendeantenne ausgerichtet sind.

Als Navigationshilfen dienen dem Arzt zusätzlich an der Ortungsantennenanordnung angebrachte optische Signalmittel, bspw. in Form illuminierbarer Pfeilsymbole, deren Illumination auf der Grundlage von jeweils in den drei Luftspulen induktiv eingekoppelten, elektrischen Energieanteilen vorgenommen wird. Ist die von allen drei Luftspulen empfangene elektrische Energie gleich groß, so befindet sich die Implantat-Ortungsantennenanordnung konzentrisch über der implantatseitig angebrachten Sendeantenne. Im Zentrum der Implant-Ortungsantennenanordnung ist eine Durchtrittsführung zum Durchstoßen einer Injektionsnadel angebracht, so dass der Arzt das darunterliegende Septum zum Zwecke der Befüllung des Medikamentenreservoirs lokal durchstechen kann.

Die Druckschrift US 2008/028127 A1 beschreibt eine Vorrichtung zur transkutanen Lokalisation eines intrakorporal platzierten Implantates, das eine extrakorporal handhabbare Implantat-Ortungsantennenanordnung vorsieht, die zum Auffinden einer intrakorporal platzierten Spule längs der Hautoberfläche verschiebbar ist, gleichsam dem vorstehend erläuterten Ortungssystem gemäß der Druckschrift EP 1 052 935 B1.

Die Druckschrift US 2017/0100056 A1 offenbart ein Kommunikationssystem zwischen einer intrakorporal platzierten Spulenanordnung und einer extrakorporal beweglich angeordneten Spulenarrayanordnung, die Signaldaten sowie Energie zwischen einer einzelnen extrakorporalen Spule und einer intrakorporal platzierten Spule zu übertragen vermag. Hierzu verfügt die intrakorporale Spule über einen größeren Spulendurchmesser als die extrakorporalen, in einer Arrayanordnung vorgesehenen Einzelspulen.

### Darstellung der Erfindung

Der Erfindung liegt die Aufgabe zugrunde, eine Vorrichtung zur transkutanen Lokalisation eines intrakorporal, subkutan verorteten medizinischen Implantats, das eine Implantatoberfläche aufweist, an der wenigstens eine erste elektrische Spule angeordnet ist, die wenigstens eine, einen Bereich der Implantatoberfläche umschließende Spulenwicklung aufweist, sowie mit einer extrakorporal handhabbaren Einheit, an der wenigstens eine elektrische Spule angebracht ist, die mit einer elektrischen Steuereinheit sowie mit einem Signalmittel verbunden ist, derart weiterzubilden, so dass der für einen Arzt zu bewältigende zeitliche Aufwand sowie auch die erforderliche Konzentrationsleistung zum manuellen Zentrieren der extrakorporal handhabbaren Einheit relativ zur implantatseitig angebrachten elektrischen Spule minimiert werden sollen. Zugleich gilt es dafür zu sorgen, dass eine nachfolgende Punktion mit Hilfe einer Injektionsnadel zuverlässig und Treffsicher durch die Haut und den Innenbereich der implantatseitig angebrachten elektrischen Spule durchgeführt werden kann. Insbesondere gilt es einerseits den Arzt zur Durchführung der vorstehend genannten Maßnahme zu entlasten und andererseits den Patienten vor möglichen Fehlpunktationen zu schützen.

Die Lösung der der Erfindung zugrunde liegenden Aufgabe ist im Anspruch 1 angegeben. Die lösungsgemäße Vorrichtung in bevorzugter Weise weiterbildende Merkmale sind in den Ansprüchen angegeben sowie der weiteren Beschreibung unter Bezugnahme auf die Ausführungsbeispiele zu entnehmen.

Die lösungsgemäße Vorrichtung zur transkutanen Lokalisation eines intrakorporal, subkutan verorteten medizinischen Implantates gemäß den Merkmalen des Oberbegriffes des Anspruches 1 zeichnet sich dadurch aus, dass die extrakorporal handhabbare Einheit eine Vielzahl von weiteren elektrischen Spulen aufweist, deren Spulendurchmesser gleichsam dem Spulendurchmesser der wenigstens einen Spule gleich oder kleiner einem der implantatseitig angebrachten elektrischen Spule zugeordneten Spulendurchmesser bemessen sind. Zudem sind die auf der extrakorporal handhabbaren Einheit angebrachten elektrischen Spulen jeweils mit einem Leuchtmittel verbunden, das zumindest bei einer maximalen induktiven Kopplung zwischen wenigstens einer der elektrischen Spulen und der implantatseitig angebrachten elektrischen Spule ein wahrnehmbares, wenigstens die elektrische Spule mit maximaler induktiver Kopplung identifizierendes Signal erzeugt.

Die lösungsgemäße Vorrichtung vermeidet den zum Teil zeitraubenden Suchvorgang für den Arzt zum Auffinden der exakten Position der implantatseitig angebrachten elektrischen Spule, bei dem der Arzt durch kontrollierte Relativbewegungen der bekannten extrakorporal handhabbaren Einheit unter Beachtung von für ihn wahrnehmbaren, vorzugsweise optischen Navigationsinformationen einen hochpräzisen Zentriervorgang durchzuführen hat. Die lösungsgemäße Idee sieht vielmehr ein Flächensubstrat in Form einer Matte oder Folie als extrakorporal handhabbare Einheit vor, die aus einem hautverträglichen und flexiblen, vorzugsweise lichttransparenten Material besteht, in dem eine Vielzahl elektrischer Spulen mit einer möglichst hohen räumlichen Packungsdichte angeordnet ist.

Vorzugsweise sind die elektrischen Spulen an oder innerhalb des mattenförmigen Flächensubstrates längs einer ersten Anordnungsebene mittelbar oder vorzugsweise unmittelbar nebeneinanderliegend angrenzend angeordnet, so dass der Flächenanteil innerhalb der ersten Anordnungsebene der jeweils nicht von wenigstens einer elektrischen Spule umfasst ist möglichst klein, vorzugsweise minimiert ist.

Die Flächenform und -größe des matten- oder folienartig ausgebildeten Flächensubstrates, in dem die Vielzahl der elektrischen Spulen zumindest bereichsweise gleich verteilt und möglichst homogen flächenabdeckend angeordnet ist, sind vorzugsweise derart gewählt, so dass durch bloßes An- bzw. Auflegen des Flächensubstrates an der Hautoberfläche in einem Bereich einer sehr wahrscheinlichen Position der subkutan verorteten implantatseitig angebrachten elektrischen Spule, wenigstens eine elektrische Spule der Vielzahl innerhalb des Flächensubstrates angebrachten elektrischen Spulen in Projektion über der implantatseitig verorteten elektrischen Spule zu liegen kommt.

Zum Zwecke der exakten Lokalisation der subkutan verorteten implantatseitig angebrachten elektrischen Spule vergleicht die ebenfalls mittel- oder unmittelbar am mattenförmigen Flächensubstrat angebrachte Steuereinheit die im Wege der Induktion in allen Flächensubstrat-seitig angebrachten elektrischen Spulen eingekoppelten elektrischen Energien und bestimmt jene elektrische Spule oder jene, aus mehreren elektrischen Spulen zusammengesetzte Spulengruppe, in der jeweils die maximale elektrische Energie im Wege einer maximalen induktiven Kopplung durch maximale Überlappung mit der Implantat-seitigen Spule messbar ist.

Mit Hilfe eines Leuchtmittels als Signalmittel, wird jene elektrische Spule bzw. Spulengruppe unmittelbar optisch markiert, die mit der implantierten elektrischen Spule maximal induktiv koppelt. Jede elektrische Spule ist innerhalb des mattenartigen Flächensubstrates mit einem Leuchtmittel versehen, so dass bei Illuminatin eines der Leuchtmittel eine wahrnehmbare Spulen-Identifikation möglich ist. Vorzugsweise ist das Leuchtmittel in Form einer LED oder eines fluoreszierenden oder phosphoreszierenden Materials ausgebildet, die bzw. das unmittelbar an jeder elektrischen Spule angebracht oder im Spulenmaterial eingebracht ist.

Das an jeder der Vielzahl der elektrischen Spulen jeweils angebrachte Leuchtmittel emittiert vorzugsweise elektromagnetische Strahlen mit Wellenlängen im sichtbaren Spektralbereich. Aus diesem Grunde ist das hautverträgliche Material, aus dem das mattenförmige Flächensubstrat gefertigt ist und die Vielzahl der elektrischen Spulen zumindest teilweise in Form einer Matrix umgibt, aus einem lichttransparenten Material, vorzugsweise Polyamid oder einem auf Silikonbasis beruhenden Kunststoffmaterial gefertigt. Das sich an die Körperoberflächenform flexibel anschmiegende Flächensubstrat ermöglicht überdies ein weitgehend ungehindertes Durchstechen mit einer Injektionsnadel, so dass nach Identifikation wenigstens einer elektrischen Spule mit maximaler induktiver Kopplung zur implantatseitig angeordneten elektrischen Spule, ein Arzt eine Injektionsnadel möglichst mittig und ungehindert innerhalb eines Kreisflächenbereiches, der von der jeweils mit Hilfe des Leuchtmittels gekennzeichneten elektrischen Spule umgeben ist, durch das Flächensubstrat sowie das in Projektion unmittelbar unter der Hautoberfläche befindliche Septum zum Zwecke einer Befüllung durchstoßen kann.

Um zu gewährleisten, dass die jeweils mit maximaler induktiver Kopplung markierte Spule in Projektion zur Hautoberfläche stets über der implantatseitig angebrachten elektrischen Spule angeordnet ist, ist der Spulendurchmesser der jeweils am Flächensubstrat angebrachten elektrischen Spulen maximal gleich, vorzugsweise kleiner als der Spulendurchmesser der implantatseitig angebrachten elektrischen Spule gewählt. Sämtliche flächensubstratseitig angeordneten elektrischen Spulen verfügen über einen gleich dimensionierten Spulendurchmesser.

Ein weiteres bevorzugtes Ausführungsbeispiel zur Ausbildung der extrakorporal handhabbare Einheit sieht wenigstens einen zweiten Anteil an elektrischen Spulen innerhalb des mattenartig ausgebildeten Flächensubstrates vor, die längs einer zur ersten Anordnungsebene parallelen zweiten Anordnungsebene nebeneinander mittel- oder unmittelbar angrenzend oder sich jeweils paarweise teilweise überlappend derart angeordnet sind, so dass sich in orthogonaler Projektion auf die erste und zweite Anordnungsebene die elektrischen Spulen beider Anordnungsebenen jeweils teilweise gegenseitig überlappen. Hierdurch wird sichergestellt, dass bei induktiver Kopplung zwischen einer der Vielzahl an elektrischen Spulen und der implantatseitig angebrachten elektrischen Spule eben jene flächensubstratseitig angeordnete elektrische Spule, die aufgrund ihrer maximalen induktiven Kopplung vorzugsweise optisch gegenüber allen anderen elektrischen Spulen wahrnehmbar in Erscheinung tritt, exakt über oder innerhalb der implantatseitig angeordneten elektrischen Spule positioniert ist.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Darstellung eines mattenartigen Flächensubstrates mit einer Vielzahl elektrischer Spulen sowie einer implantatseitig angebrachten elektrischen Spule sowie
- Fig. 2: Ausführungsbeispiel eines mattenartig ausgebildeten Flächensubstrates mit in zwei Anordnungsebenen verteilt angebrachten elektrischen Spulen.

### Wege zur Ausführung der Erfindung, gewerbliche Verwendbarkeit

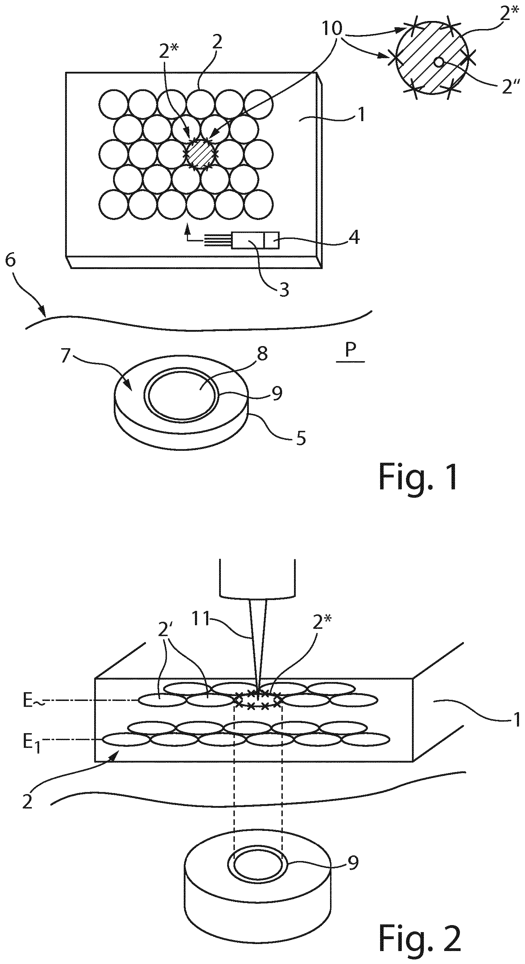

Figur 1 illustriert ein mattenartig ausgebildetes Flächensubstrat 1, das aus einem flexiblen, hautverträglichen Material gefertigt ist, vorzugsweise aus einem silikonbasierten Kunststoff, in dem eine Vielzahl gleichförmig und gleich groß dimensionierter elektrischer Spulen 2 integriert ist. Sämtliche elektrische Spulen 2 sind elektrisch mit einer Steuereinheit 3 verbunden, die mittel- oder unmittelbar mit einer elektrischen Energiequelle, vorzugsweise in Form einer Batterie oder eines Akkus 4 verbunden ist. Die elektrischen Spulen 2 können jeweils unmittelbar aneinander angrenzend längs einer Anordnungsebene innerhalb oder auf dem Flächensubstrat 1 angeordnet sein, wie diese in Figur 1 zu entnehmen ist. Hierbei bilden sich Zwischenbereiche zwischen den Spulen aus, die nicht von den elektrischen Spulen umfasst sind. Um derartige Zwischenbereiche zu minimieren oder möglichst vollständig zu beseitigen, bietet es sich alternativ an die elektrischen Spulen 2 mit einem gegenseitigem Überlapp längs einer Anordnungsebene anzuordnen.

Das mattenartig ausgebildete Flächensubstrat 1 wird zu Zwecken der Lokalisierung eines Implantats 5 auf die Hautoberfläche 6 eines Patienten P aufgelegt. Bei dem medizinischen Implantat 5 handelt es sich insbesondere um eine Medikamentenpumpenanordnung, die eine Implantatoberfläche 7 aufweist, in der wenigstens ein selbstabdichtender Port 8 in Form eines Septums eingebracht ist, das ein innerhalb des medizinischen Implantates befindliches Reservoirvolumen fluiddicht abdichtet. Im implantierten Zustand ist die Implantatoberfläche 7 weitgehend parallel zur Hautoberfläche 6 orientiert, so dass das Septum 8 nahe der Hautoberfläche 6 angeordnet ist. Zusätzlich ist das Septum 8 von einer elektrischen Spule 9 umfasst, die über eine innerhalb des medizinischen Implantates 5 enthaltende, nicht weiter dargestellte Steuereinheit sowie auch elektrische Energiequelle in Form einer Sendeantenne aktivierbar ist.

Zum Zwecke der Lokalisierung des Septums 8 wird das in Figur 1 illustrierte mattenartig ausgebildete Flächensubstrat 1 flächendeckend und konturgetreu an die Hautoberfläche 6 des Patienten P im Bereich des medizinischen Implantates 5 aufgelegt. Die Flächengrößen des Flächensubstrates 1 sowie der flächigen Anordnung und Ausdehnung der Vielzahl an Spulen 2 sind derart gewählt, so dass ein Arzt auch bei einer nur ungenauen Kenntnis über die Lage des Implantats 5 das Flächensubstrat 1 mit einer geometrischen Überlappung wenigstens einer Spule 2 mit der implantatseitig angeordneten Spule 9 auf die Hautoberfläche des Patienten zur Anlagen bringen kann ohne dabei die Position des Flächensubstrats nachträglich korrigieren zu müssen.

Die Vielzahl der an dem mattenartigen Flächensubstrat 1 angeordneten elektrischen Spulen 2 dienen jeweils als Empfangsspulen, die im Weg einer induktiven Kopplung elektrische Energie zu empfangen und aufnehmen vermögen, die mit Hilfe der Steuereinheit 3 erfasst, gemessen und zwischen den einzelnen elektrischen Spulen verglichen werden kann. Aus der Vielzahl der elektrischen Spulen 2 wird jene Spule ermittelt, die mit der implantatseitig angeordneten elektrischen Spule 9 induktiv maximal koppelt und somit über die maximale elektrische Energie verfügt. Alternativ können die am mattenartigen Flächensubstrat angeordneten elektrischen Spulen aktiv, d.h. als Sendespulen und die wenigstens eine implantatseitig angeordnete elektrische Spule passiv betrieben werden. Auf diese Weise wird die implantatseitige elektrische Energiequelle für den Lokalisationsvorgang nicht belastet. Auch in diesem Fall wird jene am mattenartigen Flächensubstrat angebrachte elektrische Spule optisch markiert, die eine maximale induktive Kopplung mit der implantatseitigen elektrischen Spule besitzt.

Jede der in dem mattenartigen Flächensubstrat 1 integrierten elektrischen Spulen 2 ist separat mit einem Leuchtmittel 10, bspw. in Form eines LEDs ausgestattet. Zur Markierung und visuellen Wahrnehmung jener elektrischen Spule 2* mit maximaler induktiver Kopplung unter allen vorhandenen elektrischen Spulen, wird das Leuchtmittel 10 eben jener elektrischen Spule 2* mit Hilfe der Steuereinheit 3 aktiviert, so dass ein Arzt anhand der jeweils aufleuchtenden elektrischen Spule 2* jenen Flächensubstratbereich schnell und eindeutig zu erfassen vermag, durch den er die Injektionsnadel zu treiben hat, um treffsicher das unmittelbar darunter befindliche Septum 8 punktieren zu können. In Figur 1 ist die beleuchtete Spule 2* mit angedeuteten Leuchtmittel 10 separat vergrößert dargestellt.

Vorzugsweise gilt es die Injektionsnadel mittig innerhalb der Kreisfläche 2" zu positionieren, die von der jeweils aufleuchtenden elektrischen Spule 2 radial begrenzt wird.

In Figur 2 ist ein Ausführungsbeispiel eines mattenartig ausgebildeten Flächensubstrates 1 dargestellt, bei dem längs einer ersten Anordnungsebene E1 eine Vielzahl elektrischer Spulen 2 angeordnet ist, vergleichbar das in Figur 1 dargestellte Ausführungsbeispiel, d. h. die in der ersten Anordnungsebene E1 angeordneten elektrischen Spulen 2 sind gleich groß und arrayförmig unmittelbar einander anliegend angeordnet. Ferner ist eine weitere Vielzahl von elektrischen Spulen 2' in einer zweiten Anordnungsebene E2 angeordnet, die parallel zur ersten Anordnungsebene E1 orientiert ist, wobei die Spulen 2' innerhalb der zweiten Anordnungsebene E2 um jeweils einen Spulenhalbdurchmesser gegenüber der Spulen 2 innerhalb der ersten Anordnungsebene E1 versetzt angeordnet sind. In Projektion orthogonal zu beiden Anordnungsebenen E1, E2 überlappen demzufolge die elektrischen Spulen 2, 2' aus den jeweils beiden unterschiedlichen Anordnungsebenen E1, E2. Auf diese Weise ist die Sicherheit erhöht, mit der wenigstens eine Spule exakt orthogonal über und innerhalb der implantatseitig angebrachten elektrischen Spule 9 zur Anlage gebracht werden kann, so dass ein Arzt beim Durchstechen des Innenbereiches der mit Hilfe eines geeigneten Leuchtmittels illuminierten elektrischen Spule 2* exakt das unmittelbar intrakorporal darunter befindliche Septum 9 des Implantats mit Hilfe einer Injektionsnadel 11 punktiert.

Die lösungsgemäße Vorrichtung zur transkutanen Lokalisation eines intrakorporal subkutan verordneten medizinischen Implantates vermeidet jegliche Suchbewegungen und gestattet durch einmaliges Auflegen des mit einer Vielzahl von elektrischen Spulen versehenen, mattenartig ausgebildeten Flächensubstrates auf die Hautoberfläche eine sofortige Anzeige und Identifikation jener elektrischen Spulen, die sich über dem intrakorporal verorteten Septum befindet. Vorzugsweise weist das Flächensubstrat eine möglichst gegen Verrutschen an der Hautoberfläche konditionierte Oberfläche auf, bspw. geeignet zur Ausbildung einer Haftmittel-freien adhäsiven Oberflächenfügung.

### Bezugszeichenliste

- 1: mattenartig ausgebildetes Flächensubstrat
- 2: elektrische Spulen
- 2': Elektrische Spulen der zweiten Anordnungsebene
- 2*: illuminierte elektrische Spule
- 3: Steuereinheit
- 4: elektrische Energiequelle
- 5: medizinisches Implantat
- 6: Hautoberfläche
- 7: Implantatoberfläche
- 8: Septum
- 9: implantatseitig angeordnete elektrische Spule
- 10: Leuchtmittel
- 11: Injektionsnadel
- E1, E2: erste und zweite Anordnungsebene

## Patentansprüche

1. Vorrichtung zur transkutanen Lokalisation eines intrakorporal, subkutan verorteten medizinischen Implantats (5), umfassend das eine Implantatoberfläche (7) aufweisende, medizinische Implantat (5), an der wenigstens eine erste elektrische Spule (9) angeordnet ist, die wenigstens eine, einen Bereich der Implantatoberfläche (7) umschließende Spulenwicklung aufweist, sowie mit einer extrakorporal handhabbaren Einheit, an der wenigstens eine elektrische Spule (2) angebracht ist, die mit einer elektrischen Steuereinheit (3) sowie mit einem Leuchtmittel verbunden ist,
wobei die in Form eines Flächensubstrats (1) ausgebildete extrakorporal handhabbare Einheit eine Vielzahl von weiteren elektrischen Spulen (2) aufweist, deren Spulendurchmesser gleichsam dem Spulendurchmesser der wenigstens einen Spule (2) gleich oder kleiner einem der Implantat-seitig angebrachten elektrischen Spule (9) zugeordneten Spulendurchmesser bemessen sind,
**dadurch gekennzeichnet, dass** die auf oder an dem Flächensubstrat (1) angebrachten elektrischen Spulen (2) mit der elektrischen Steuereinheit (3) sowie mit jeweils einem der Leuchtmittel (10) verbunden und derart angeordnet und ausgebildet sind, dass zumindest bei einer maximalen induktiven Kopplung zwischen wenigstens einer der an der extrakorporal handhabbaren Einheit angebrachten elektrischen Spulen (2) und der wenigstens einen Implantat-seitig angebrachten elektrischen Spule (9) durch Illumination des Leuchtmittels (10) wenigstens jener elektrischen Spule (2*) mit maximaler induktiver Kopplung eine von Außen wahrnehmbare Spulen-Identifikation möglich ist.

2. Vorrichtung nach Anspruch 1,
**dadurch gekennzeichnet, dass** wenigstens ein erster Anteil der an der extrakorporal handhabbaren Einheit angebrachten elektrischen Spulen (2) längs einer ersten Anordnungsebene (E1) nebeneinander mittel- oder unmittelbar angrenzend oder sich jeweils paarweise teilweise überlappend angeordnet ist.

3. Vorrichtung nach Anspruch 2,
**dadurch gekennzeichnet, dass** wenigstens ein zweiter Anteil der an der extrakorporal handhabbaren Einheit angebrachten elektrischen Spulen (2') längs einer zur ersten Anordnungsebene (E1) parallelen zweiten Anordnungsebene (E2) nebeneinander mittel- oder unmittelbar angrenzend oder sich jeweils paarweise teilweise überlappend derart angeordnet ist, so dass sich in orthogonaler Projektion auf die erste und zweite Anordnungsebene (E1, E2) die elektrischen Spulen (2, 2') beider Anordnungsebenen (E1, E2) jeweils teilweise gegenseitig überlappen.

4. Vorrichtung nach einem der Ansprüche 1 bis 3,
**dadurch gekennzeichnet, dass** das Leuchtmittel (10) eine LED, fluoreszierendes oder phosphoreszierendes Material aufweist.

5. Vorrichtung nach einem der Ansprüche 1 bis 4,
**dadurch gekennzeichnet, dass** das medizinische Implantat (5) wenigstens ein Reservoirvolumen aufweist, das zumindest von dem wenigstens einen, von der Spulenwicklung umschlossenen Bereich der Implantatoberfläche (7) begrenzt ist, und
dass der Bereich als selbstabdichtender Port zum Befüllen mittels einer Injektionsvorrichtung (11) ausgebildet ist.

6. Vorrichtung nach Anspruch 5,
**dadurch gekennzeichnet, dass** der Bereich in Art eines selbstabdichtenden Septums (8) ausgebildet ist.

## Claims

1. Device for transcutaneously locating an intracorporeal, subcutaneously located medical implant (5), comprising a medical implant (5) having an implant surface (7), on which at least one first electrical coil (9) is arranged, which comprises at least one coil winding surrounding one area of the implant surface (7), as well as with an extracorporeally operable unit, to which at least one electrical coil (2) is applied, which is connected to an electrical control unit (3) as well as a lamp,
wherein the extracorporeally operable unit designed in the form of a surface substrate (1) comprises a plurality of further electrical coils (2), the coil diameters of which like the coil diameter of the at least one coil (2) are dimensioned to be equal to or smaller than a coil diameter assigned to an electrical coil (9) on the implant side,
**characterised in that** the electrical coils (2) applied onto or on the surface substrate (1) are each connected to the electrical control unit (3) and to one of the lamps (10) and are arranged and designed in such a way that at least in the case of a maximum inductive coupling between at least one of the electrical coils (2) applied on the extracorporeally operable unit and the at least one of the electrical coils (9) applied on the implant side, through illumination of the lamp (10) of at least the electrical coil (2*) with maximum inductive coupling, externally visible coil identification is possible.

2. Device according to claim 1,
**characterised in that** at least a first portion of the electrical coils (2) applied on the extracorporeally operable unit is arranged along a first arrangement plane (E1) next to each other in a directly or indirectly adjoining manner or partially overlapping each other in pairs.

3. Device according to claim 2,
**characterised in that** at least a second portion of the electrical coils (2') applied on the extracorporeally operable unit is arranged along second arrangement plant (E2) parallel to the first arrangement plane (E1) next to each other in a directly or indirectly adjoining manner or partially overlapping each other in pairs in such a way that in orthogonal projection onto the first and second arrangement plane (E1, E2), the electrical coils (2, 2') of both arrangement planes (E1, E2) partially overlap each other.

4. Device according to any one of claims 1 to 3,
**characterised in that** the lamp (10) comprises an LED, fluorescent of phosphorescent material.

5. Device according to any one of claims 1 to 4,
**characterised in that** the medical implant (5) comprises at least one reservoir volume that is delimited by the at least one area of the implant surface (7) surrounded by the coil winding, and
**in that** the area is designed as a self-sealing port for filling by means of an injection device (11).

6. Device according to claim 5,
**characterised in that** the area is designed in the form of a self-sealing septum (8).

## Revendications

1. Dispositif de localisation transcutanée d'un implant médical (5) localisé de façon intracorporelle, sous-cutanée, comprenant l'implant médical (5) comportant une surface d'implant (7) sur laquelle est disposée au moins une première bobine électrique (9), qui comporte au moins un enroulement de bobine entourant une zone de la surface d'implant (7), ainsi qu'avec une unité extra-corporellement manipulable sur laquelle est placée au moins une bobine électrique (2), qui est reliée à une unité de commande électrique (3) ainsi qu'à un moyen d'éclairage,
sachant que l'unité extra-corporellement, manipulable constituée sous la forme d'un substrat superficiel (1) comprend une pluralité d'autres bobines électriques (2), dont les diamètres de bobine tout comme le diamètre de bobine d'au moins une bobine (2) sont dimensionnés identiques ou plus petits qu'un diamètre de bobine attribué à la bobine électrique (9) placée côté implant,
**caractérisé en ce que** les bobines électriques (2) placées sur ou près du substrat superficiel (1) sont reliées à l'unité de commande électrique (3) ainsi respectivement qu'à un des moyens d'éclairage (10) et sont disposées et constituées de telle manière qu'au moins une identification de bobine perceptible de l'extérieur est possible au moins lors d'un couplage inductif maximal entre au moins une des bobines électriques (2) placées sur l'unité extra-corporellement manipulables et au moins une bobine électrique (9) placée côté implant par illumination du moyen d'éclairage (10) au moins de chaque bobine électrique (2*) avec un couplage inductif maximal.

2. Dispositif selon la revendication 1,
**caractérisé en ce qu'**au moins une première partie des bobines électriques (2) placées sur l'unité extracorporelle manipulable est disposée le long d'un premier plan de montage (E1) l'une à côté de l'autre directement ou indirectement limitrophes ou se chevauchant respectivement en partie par paires.

3. Dispositif selon la revendication 2,
**caractérisé en ce qu'**au moins une deuxième partie des bobines électriques (2') placées sur l'unité extra-corporellement manipulable est disposée le long d'un deuxième plan de montage (E2) parallèle au premier plan de montage (E1) l'une à côté de l'autre directement ou indirectement limitrophes ou se chevauchant respectivement en partie par paires de telle manière que les bobines électriques (2, 2') des deux plans de montage (E1, E2) se chevauchent respectivement en partie réciproquement en projection orthogonale sur le premier et le deuxième plan de montage (E1, E2).

4. Dispositif selon l'une quelconque des revendications 1 à 3,
**caractérisé en ce que** le moyen d'éclairage (10) comporte un matériau à DEL [diodes électroluminescentes], fluorescent ou phosphorescent.

5. Dispositif selon l'une quelconque des revendications 1 à 4,
**caractérisé en ce que** l'implant médical (5) comporte au moins un volume de réservoir, qui est limité au moins par au moins une zone de la surface d'implant (7), entourée par l'enroulement de bobine, et
**en ce que** la zone est constituée sur la forme d'un orifice auto-étanchéifiant pour le remplissage au moyen d'un dispositif d'injection (11).

6. Dispositif selon la revendication 5,
**caractérisé en ce que** la zone est constituée sous la forme d'un septum auto-étanchéifiant (8).
